# EUROPÄISCHE PATENTSCHRIFT

(11) **EP 3 937 801 B1**
(45) Veröffentlichungstag und Bekanntmachung des Hinweises auf die Patenterteilung: **19.06.2024**
(21) Anmeldenummer: 20710111.4
(22) Anmeldetag: 05.03.2020
(51) Int. Cl.: A61B 17/28, A61B 17/32

(54) **CHIRURGISCHES INSTRUMENT MIT DURCHSTRÖMBAREM SCHLUSSBEREICH**
SURGICAL INSTRUMENT HAVING TERMINAL REGION THROUGH WHICH FLOW CAN OCCUR
INSTRUMENT CHIRURGICAL COMPORTANT UNE ZONE DE FERMETURE PERMETTANT UN ÉCOULEMENT TRANSVERSAL

(30) Priorität: 14.03.2019 DE 102019106512
(43) Veröffentlichungstag der Anmeldung: 19.01.2022
(73) Patentinhaber: Aesculap AG, 78532 Tuttlingen (DE)
(72) Erfinder: BARTHELMES, Sven, 78576 Emmingen-Lipptingen (DE); SCHABERT, Stefanie, 78554 Aldingen (DE)
(74) Vertreter: Winter, Brandl - Partnerschaft mbB
(86) Internationale Anmeldenummer: PCT/EP2020/055910
(87) Internationale Veröffentlichungsnummer: WO 2020/182625

(56) Entgegenhaltungen:
- DE-A1- 2 061 539
- DE-A1-102016 111 892
- DE-U1- 8 316 305
- US-A1- 2007 276 431

## Beschreibung

Die Erfindung betrifft ein chirurgisches Instrument der branchenüberkreuzten Bauart mit einer männlichen Branche und einer weiblichen Branche, die in einer Montageposition des Instruments über einen Durchsteckschluss schwenkbar aneinander gehalten sind, wozu dessen weibliche Branche in ihrem Schlussbereich eine Durchstecköffnung ausbildet, in der ein Schlussbereich der männlichen Branche schwenkbar aufgenommen werden kann.

### Hintergrund der Erfindung

Bei der Vorbereitung und Durchführung von chirurgischen Behandlungen kommen je nach Behandlung verschiedene chirurgische Instrumente zum Einsatz, darunter beispielsweise Klemmen zum Befestigen von OP-Tüchern, Kabeln oder Schläuchen und Zangen für unterschiedliche Anwendungen. Klemmen und Zangen sind üblicherweise Instrumente mit zwei sich überkreuzenden Branchen, ähnlich der Scherenbauart, und sind in der Montageposition in ihrem Schlussbereich aneinander anscharniert und/oder über einen Durchsteckschluss aneinander gehalten.

Nach der Behandlung bzw. vor deren Einsatz müssen sämtliche Instrumente gereinigt und sterilisiert werden. Dabei ist insbesondere darauf zu achten, dass die Reinigung / Sterilisation unbedingt an allen zugänglichen Stellen des Instruments erfolgt, um eine maximale Patientensicherheit zu gewährleisten. Dazu werden Instrumente mit zwei sich überkreuzenden Branchen üblicherweise in ihre maximal geöffnete Position gebracht bevor sie dann dem weiteren Reinigungs-/Sterilisationsverfahren unterzogen werden, beispielsweise in einen Sterilcontainer eingebracht und unter Hitzeeinwirkung heißsterilisiert oder chemisch in einem Reinigungs-/Sterilisationsbad behandelt werden.

### Stand der Technik

Chirurgische Instrumente der branchenüberkreuzten Bauart, beispielsweise Klemmen oder Zangen, die über einen Durchsteckschluss miteinander verbunden sind, sind bereits bekannt. Dabei weisen die beiden Branchen jeweils einen Schlussbereich auf, der in Längsrichtung der Branchen gesehen zwischen einem distalen Effektorabschnitt (bspw. Greiffläche) und einem proximalen Greifabschnitt angeordnet ist. Der Schlussbereich der weiblichen Branche weist eine längliche Durchgangsöffnung auf, die dazu vorgesehen ist, den Schlussbereich der männlichen Branche aufzunehmen. Zur Montage wird die Durchgangsöffnung zunächst aufgeweitet, beispielsweise mit Hilfe eines Dorns, um die männliche Branchen durchzuführen, sodass sich der Schlussbereich der männlichen Branche in der Durchgangsöffnung befindet. Anschließend wird der aufgeweitete Schlussbereich in seine Ausgangsform zurück verformt. Zur Anscharnierung der beiden Branchen aneinander kann entweder ein Bolzen-Lageröffnungs-Mechanismus an den einander zugewandten Wänden der Schlussbereiche angeordnet sein, oder die Schlussbereiche der beiden Branchen weisen ein koaxiales Durchgangsloch auf, in die ein separater Sicherungsstift eingeführt wird, um dessen Längsachse die beiden Branchen dann drehbar aneinander gesichert sind. Alternativ kann der Schlussbereich auch wie in der DE 10 2017 105 70 A1 mit teilkreisförmigen Nuten und Vorsprüngen ausgestaltet sein.

Eine aus dem Stand der Technik bekannte chirurgische Zange ist beispielsweise in der deutschen Patenanmeldung DE 2061 539 offenbart mit zwei Branchen, die über einen Durchsteckschluss miteinander verbunden sind, wobei an den einander gegenüberliegenden Innenflächen der Durchstecköffnung der weiblichen Branche jeweils ein Vorsprung vorgesehen ist, welche koaxial zueinander angeordnet sind und jeweils in eine komplementär ausgebildete Aussparung am Schlussbereich der männlichen Branche greifen, um diesen schwenkbar in der Durchstecköffnung zu halten.

Die internationale Patentanmeldung WO 2016/169037 A1 offenbart ebenfalls eine chirurgische Zange mit zwei Branchen, die über einen Durchsteckschluss aneinander gehalten sind. Weitere Beispiele eines chirurgischen Instruments mit zwei Branchen, welche über einen Durchsteckschluss miteinander verbunden sind, sind in DE 10 2016 111 892 A1, DE 83 16 305 U1 und US 2007 / 0 276 431 A1 offenbart.

Aus Stabilitätsgründen ist die Durchgangsöffnung im Schlussbereich der weiblichen Branche üblicherweise mit einer Länge ausgeführt, in der der Schlussbereich der männlichen Branche in der vollständig geschlossenen Position des Instruments gerade so aufgenommen werden kann. Dies hat zur Folge, dass in vollständig geöffneter Position des Instruments die Außenfläche der männlichen Branche so an der Außenfläche der weiblichen Branche anliegt, dass die Längsenden der Durchgangsöffnung auf den der männlichen Branche zugewandten Seiten von der Außenfläche der männlichen Branche verdeckt werden bzw. die der männlichen Branche zuwandten Längskanten der Durchgangsöffnung an der Außenfläche der männlichen Branche anliegen. Auf diese Weise bilden sich im Bereich der Durchgangsöffnung in der maximalen Öffnungsposition des Instruments zwei Taschen, nämlich entlang der Innenwand der Durchgangsöffnung und der die Durchgangsöffnung verschließenden Außenfläche der männlichen Branche. Diese Taschen können nicht durchströmt werden, was zu einer erschwerten Reinigung und/oder Sterilisation führen kann.

Die Reinigung dieser Instrumente stellt ebenfalls eine bekannte Herausforderung dar. Die deutsche Patentanmeldung DE 10 2016 111 892 A1 beschreibt eine chirurgische Zange mit zwei Branchen, die über einen Durchsteckschluss aneinander gehalten sind und deren Kontaktflächen im Schlussbereich jeweils eine zueinander unterschiedliche Oberflächenstruktur aufweisen, sodass der Kontakt beider Instrumentenbranchen aneinander linien- und/oder punktförmig ist und sich zwischen den Kontaktlinien / Kontaktpunkten Freiräume bilden, die für ein Desinfektionsmittel erreichbar sind. Die Durchströmbarkeit dieser Ausgestaltung ist jedoch nicht ausreichend.

Der Stand der Technik hat somit immer den Nachteil einer mangelhaften Desinfizierbarkeit bzw. Reinigungsmöglichkeit, was für den Patienten sowie den Anwender eine erhebliche Gesundheitsgefahr darstellt.

### Kurze Beschreibung der Erfindung

Die Aufgabe der vorliegenden Erfindung ist es somit, die Nachteile des Stands der Technik zu überwinden oder zumindest zu mildern und insbesondere ein chirurgisches Instrument der branchenüberkreuzten Bauart zu schaffen, dessen Branchen mittels eines Durchsteckschlusses aneinander gehalten sind und welches in jeder Öffnungsposition des montierten Instruments bzw. jeder Relativposition der beiden Branchen von einem Reinigungs- und/oder Sterilisationsmedium gut durchströmbar ist.

Die Aufgabe wird gelöst durch ein chirurgisches Instrument der branchenüberkreuzten Bauart mit den Merkmalen des Anspruchs 1. Vorteilhafte Weiterbildungen der Erfindung sind Gegenstand der beigefügten Unteransprüche.

Ein Grundgedanke der Erfindung besteht darin, ein chirurgisches Instrument der branchenüberkreuzten Bauart mit einer männlichen und einer weiblichen Branche zu schaffen, die mittels eines Durchsteckschlusses aneinander gehalten sind, bei dem der Durchsteckschlitz eine verbreiterte Form einnimmt.

Konkret wird ein chirurgisches Instrument der branchenüberkreuzten Bauart geschaffen mit einer weiblichen Branche und einer männlichen Branche, die über ihre Längserstreckung jeweils einen Schlussabschnitt aufweisen und der Schlussabschnitt der weiblichen Branche von einer Durchgangsöffnung durchzogen ist, die eine distale und eine proximale Längsendfläche mit jeweils zwei Außenkanten aufweist und dazu angepasst ist, den Schlussabschnitt der männlichen Branche aufzunehmen, sodass die beiden Schlussabschnitte in einer Montageposition des Instruments einen Durchsteckschluss bilden. Dabei hat die Durchgangsöffnung eine Länge, bei der die (alle) Außenkanten der Längsendflächen in allen Relativpositionen der beiden Branchen, insbesondere in einer vollständig geschlossenen Position und/oder in einer vollständig geöffneten Position des Instruments, (zumindest abschnittsweise) freiliegen und (zumindest abschnittsweise) nicht von der männliche Branche bzw. dessen Außenfläche verdeckt sind bzw. von dieser kontaktiert werden.

Insbesondere bildet die die Durchgangsöffnung weiter zwei einander (im Wesentlichen parallel) gegenüberliegende Innenkontaktflächenaus, durch die die beiden Längsendflächen miteinander verbunden sind, der Schlussabschnitt der männlichen Branche zwei voneinander abgewandte Außenkontaktflächen sowie die beiden Außenkontaktflächen miteinander verbindende Außenquerflächen ausbilden, derart, dass im Durchsteckschluss jeweils eine Innenkontaktfläche und eine Außenkontaktfläche drehbar aneinander gehalten sind, und in allen Relativpositionen der Branchen jeweils zwischen der proximalen und der distalen Längsendfläche und einer Außenquerfläche ein durchströmbarer Kanal für ein Reinigungsmittel gebildet ist.

Auf diese Weise kann sichergestellt werden, dass das chirurgische Instrument in allen Positionen der Branchen zueinander für ein Reinigungsmittel durchströmbar ist und folglich ein gesundheitliches Risiko für den Anwender und den Patienten, welches durch Restkeime bzw. nach der Reinigung verbliebene Verunreinigungen entsteht, reduziert wird. Durch die verbreiterte Abmessung bzw. erfindungsgemäße Länge der Durchgangsöffnung wird insbesondere eine Taschenbildung im maximal geöffneten Zustand des Instruments verhindert, in denen sich das Reinigungsmittel ansammelt anstatt die Brachen zu umströmen.

Unter "proximal" wird stets das zum Anwender des chirurgischen Instruments hin gerichtete Ende verstanden, unter "distal" stets das vom Anwender weg gerichtete Ende.

Bevorzugt nimmt die Durchgangsöffnung (im Längsschnitt der Branche) die Form eines Durchgangslanglochs ein. Die Längsenden der Durchgangsöffnung sind folglich abgerundet, insbesondere in Form von Halbkreisen. Besonders bevorzugt ist dabei zumindest eine Längsendfläche, vorzugsweise beide Längsendflächen, des Durchgangslanglochs jeweils zumindest zu einer seiner Öffnungsaußenseiten, bevorzugt jeweils zu beiden Öffnungsseiten, abgeschrägt, um einen Öffnungsquerschnitt an zumindest einer Öffnungsaußenseite zu vergrößern.

Der erfindungsgemäße Vorteil einer Durchströmbarkeit der Durchgangsöffnung an ihren Längsenden kann auf diese Weise weiter vergrößert werden.

Die beiden Schlussabschnitte der beiden Branchen können insbesondere jeweils zwischen einem distalen Effektorabschnitt und einem proximalen Griffabschnitt angeordnet sein, wobei (im montierten Zustand der Branchen bzw. des Instruments) die Effektorabschnitte der beiden Branchen jeweils eine der anderen Branche zugewandte Effektorinnenfläche und die Griffabschnitte der beiden Branchen jeweils eine der anderen Branche zugewandte Griffinnenfläche aufweisen. Bei den Außenquerflächen des Schlussabschnitts an der männlichen Branche kann es sich weiter um eine (schräg zur Längserstreckung der Branche verlaufende) distale Querfläche, eine (schräg zur Längserstreckung der Branche verlaufende) proximale Querfläche und zwei im Wesentlichen parallel zueinander ausgerichtete (und parallel zur Längserstreckung der Branche verlaufende) Längsflächen handeln, wobei sich die distale Querfläche an die Effektorinnenfläche der männlichen Branche anschließt und die proximale Querfläche sich an die Griffinnenfläche der männlichen Branche anschließt und die Übergänge von distaler Querfläche zur Effektorinnenfläche sowie von proximaler Querfläche zur Griffinnenfläche in Form einer distalen bzw. proximalen Ausrundung gebildet sind, welche sich über die gesamte Breite der Branchen zieht.

Unter Breite ist in diesem Zusammenhang die Erstreckungsrichtung quer zur Längsachse der Branchen und senkrecht zur Schwenkebene der Branchen zu verstehen.

Die Ausrundung am Übergang zwischen Effektorinnenfläche bzw. Griffinnenfläche und Schlussabschnitt hat den Vorteil einer verbesserten Reinigbarkeit, da sich Keime und Schmutzpartikel, im Vergleich zu etwa einer eckigen Kante, nicht festsetzen können und von Reinigungsmittel und/oder einem Reinigungsinstrument gut erfassen und ausspülen lassen.

Vorzugsweise weisen der Effektorabschnitt und der Griffabschnitt der männlichen Branche jeweils zwei voneinander abgewandte Außenflächen auf, die sich an die Außenkontaktflächen des Schlussbereichs anschließen und an ihren Übergängen zu den Außenkontaktflächen jeweils eine Stufe ausbilden, die gegenüber der Außenkontaktfläche vorspringt bzw. gegenüber der Außenkontaktfläche um einen vorbestimmten Radius versetzt ist, wobei die Stufen auf Seite der Effektorinnenfläche distal zur distalen Ausrundung und die Stufen auf Seite der Griffinnenfläche proximal zur proximalen Ausrundung angeordnet sind.

Diese geometrische Anordnung hat den Vorteil, dass eine Kerbwirkung, die an zwei "aufeinander fallenden" Kanten, beispielsweise am Übergang von distaler bzw. proximaler Querfläche zur Effektorinnenfläche bzw. Griffinnenfläche und einer Stufenkante, verhindert wird und folglich dem gesamten Instrument eine erhöhte Stabilität verliehen wird.

Insbesondere vorzugsweise weist der Schlussabschnitt der weiblichen Branche zwei im Wesentlichen parallel zu den Innenkontaktflächen verlaufende Schlussaußenflächen auf, die im geschlossenen Zustand des Instruments zu den Außenflächen des Griffabschnitts sowie des Effektorabschnitts der männlichen Branche beabstandet angeordnet sind und insbesondere auf diese Weise einen Spalt bilden.

Der auf diese Weise entstehende deutliche Spalt zwischen den Schlussaußenflächen der weiblichen Branche und dem Griff- bzw. Effektorabschnitt der männlichen Branchen ermöglicht eine verbesserte Reinigung.

### Kurzbeschreibung der Figuren

Die Erfindung wird nachstehend anhand eines bevorzugten Ausführungsbeispiels unter Bezugnahme auf die beigefügte Zeichnung näher beschrieben. Es zeigen:
Fig. 1 eine perspektivische Frontansicht eines chirurgischen Instruments in geöffnetem Zustand gemäß einer Ausführungsform der Erfindung;
Fig. 2 eine perspektivische Rückansicht der in Fig. 1 dargestellten Ausführungsform des chirurgischen Instruments in geöffnetem Zustand;
Fig. 3 eine perspektivische frontale Ansicht der in Fig. 1 abgebildeten Ausführungsform des chirurgischen Instruments in einer maximal geöffneten Position;
Fig. 4 eine perspektivische Rückansicht der in Fig. 1 abgebildeten Ausführungsform des chirurgischen Instruments in einer maximal geöffneten Position;
Fig. 5 eine Draufsicht auf die in Fig. 1 gezeigte Ausführungsform des chirurgischen Instruments in vollständig geschlossenem Zustand;
Fig. 6 eine Seitenansicht der in Fig. 1 gezeigten Ausführungsform des chirurgischen Instruments in vollständig geschlossenem Zustand.

Die Figuren sind lediglich schematischer Natur und dienen ausschließlich dem Verständnis der Erfindung. Die gleichen Elemente sind mit denselben Bezugszeichen bezeichnet.

### Detaillierte Beschreibung bevorzugter Ausführungsbeispiele

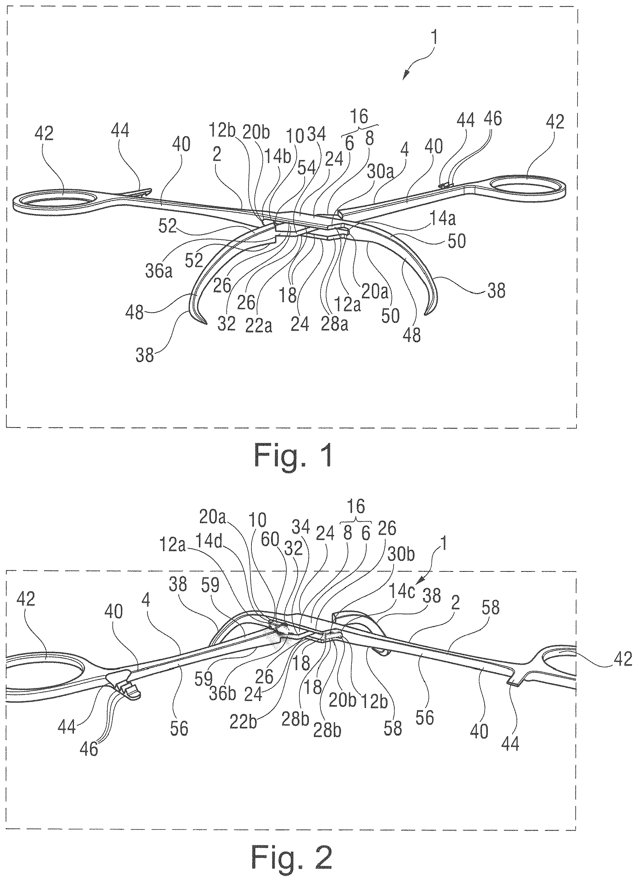

In Fig. 1 ist eine perspektivische Frontansicht eines chirurgischen Instruments 1 gemäß eine Ausführungsform der Erfindung gezeigt. Zu sehen ist ein chirurgisches Instrument 1 der branchenüberkreuzten Bauart mit einer weiblichen Branche 2 und einer männlichen Branche 4 gezeigt, die über ihre Längserstreckung jeweils einen Schlussabschnitt 6, 8 aufweisen und der Schlussabschnitt 6 der weiblichen Branche 2 von einer Durchgangsöffnung 10 durchzogen ist, die eine distale und eine proximale Längsendfläche 12a, b mit jeweils zwei Außenkanten 14a-d aufweist und dazu angepasst ist, den Schlussabschnitt 8 der männlichen Branche 4 aufzunehmen, sodass die beiden Schlussabschnitte 6, 8 in einer Montageposition des Instruments 4 einen Durchsteckschluss bilden. Dabei hat die Durchgangsöffnung 10 eine Länge, bei der die Außenkanten 14a-d der Längsendflächen 12a, b in allen Relativpositionen der beiden Branchen 2, 4, insbesondere in einer vollständig geschlossenen Position und/oder in einer vollständig geöffneten Position des Instruments 1, freiliegen und nicht von der männliche Branche 4 verdeckt sind.

Zu sehen ist insbesondere der Schlussbereich 16 des Instruments 1 in geöffnetem Zustand. Im Schlussbereich 16 des Instruments 1 befindet sich der Schlussabschnitt 8 der männlichen Branche 4 (nachfolgend als männlicher Schlussabschnitt 8 bezeichnet) in einer Durchgangsöffnung 10 im Schlussabschnitt 6 der weiblichen Branche 2 (nachfolgend als weiblicher Schlussabschnitt 2 bezeichnet) und ist in dieser drehbar gehalten. Der männliche Schlussabschnitt 8 und der weibliche Schlussabschnitt 6 bilden auf diese Weise einen Durchsteckschluss, in dem die beiden Branchen 2, 4 drehbar aneinander anscharniert sind. Die Ebene, in der die Branchen 2, 4 um das Scharnier (nicht gezeigt) des Schlussbereichs 16 des Instruments 1 relativ zueinander verschwenkt werden können, wird als Schwenkebene bezeichnet. Dabei ist die Schwenkebene eine gemeinsame Schwenkebene der beiden Branchen 2, 4.

Die Durchgangsöffnung 10 des weiblichen Schlussabschnitts 8 ist in dieser Ausführungsform ein Durchgangslangloch 10. Das Durchgangslangloch 10 weist zwei einander gegenüberliegende Innenkontaktflächen 18 auf, die im Wesentlichen parallel zur Schwenkebene verlaufen. An seinen Längsenden 20a, b weist das Durchgangsloch 10 zwei Längsendflächen 12a, b auf, über die die Innenkontaktflächen 18 miteinander verbunden sind. Die gerundeten Längsenden 20a, b des Durchganglanglochs 10 sind zu den beiden Öffnungsaußenseiten 22a, b des Durchgangslanglochs 10 (zu sehen in Fig. 1 ist nur eine erste Öffnungsaußenseite 22a, die zweite Öffnungsaußenseite 22b ist in Fig. 2 zu sehen) jeweils abgeschrägt und vergrößern jeweils den Öffnungsquerschnitt des Durchgangslanglochs 10 zu den beiden Öffnungsaußenseiten 22a, b hin. Der weibliche Schlussabschnitt 6 hat weiter zwei im Wesentlichen parallel zur Schwenkebene verlaufende Schlussaußenflächen 24, die jeweils zwei sich in Längsrichtung der Branchen 2, 4 erstreckende Längskanten 26 und zwei schräg verlaufende Querkanten 28a, b, nämlich eine distale Querkante 28a und eine proximale Querkante 28b aufweisen. Quer zu den Schlussaußenflächen 24 erstrecken sich distale und proximale Querflächen 30a, b, die von den Querkanten 28a, b aus zum Durchgangslangloch 10 hin verlaufen. Zu sehen ist weiter, dass das Durchgangslangloch 10 sich in Längsrichtung der weiblichen Branche 4 entlang des gesamten weiblichen Schlussabschnitts 6 erstreckt und die beiden Längsenden 20a, b des Durchgangslanglochs 10 die distalen und proximalen Querkanten 28a, b überschreiten, d. h. das distale Längsende 20a befindet sich distal zur distalen Querkante 28a und das proximale Längsende 20b befindet sich proximal zur proximalen Querkante 28b.

Der männliche Schlussabschnitt 8 weist zwei sich im Wesentlichen parallel zur Schwenkebene erstreckende, voneinander abgewandte Außenkontaktflächen 32 auf. Quer zu den Außenkontaktflächen 32 verlaufen am männlichen Schlussabschnitt 8 vier Querflächen 34, 36a, b, die die beiden Außenkontaktflächen 32 miteinander verbinden. Zwei von den vier Querflächen 34, 36a, b erstrecken sich im Wesentlichen in Längsrichtung der männlichen Branche 4 und werden nachfolgend als Längsflächen 34 bezeichnet. Die anderen beiden Querflächen stellen eine distale Querfläche 36a und eine proximale Querfläche 36b dar, die jeweils schräg zur Längsrichtung der männlichen Branche 4 verlaufen.

Im Schlussbereich 16 des Instruments 1 bzw. in dessen Durchsteckschluss liegen die Außenkontaktflächen 32 des männlichen Schlussabschnitts 8 an den Innenkontaktflächen 18 des weiblichen Schlussabschnitts 6 drehbar an. Das Scharnier, über das die beiden Branchen 2, 4 drehbar bzw. schwenkbar aneinander gehalten sind, kann über einen bekannten Bolzen-Aufnahmeöffnungs-Mechanismus erfolgen und wird in den Figuren nicht gezeigt. Dabei können entweder die Innenkontaktflächen 18 jeweils einen Bolzen aufweisen, die koaxial angeordnet sind und jeweils in eine Durchgangsöffnung in den Außenkontaktflächen 32 greifen, oder die Außenkontaktflächen 32 können jeweils einen Bolzen aufweisen, die koaxial angeordnet sind und jeweils in eine Durchgangsöffnung an den Innenkontaktflächen 18 greifen. Insbesondere sind die beiden Branchen über einen Niet (beispielsweise Kugel-oder Stufenniet) miteinander verbunden.

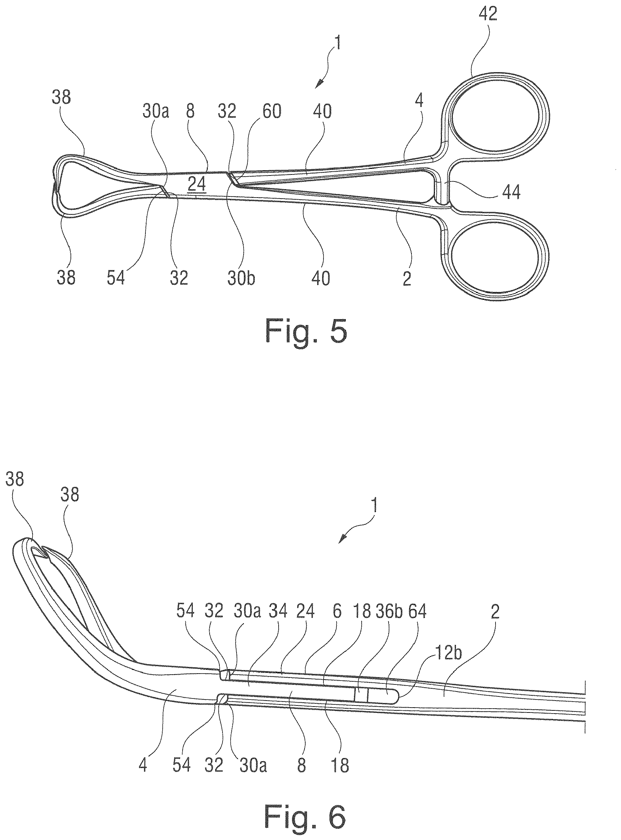

Der männliche und der weibliche Schlussabschnitt 6, 8 befinden sich jeweils zwischen einem distalen Effektorabschnitt 38 und einem proximalen Griffabschnitt 40. In der gezeigten Ausführungsform ist das chirurgische Instrument 1 eine chirurgische Klemme und die Effektorabschnitte 38 sind geschwungenen Greifteile, deren Spitzen im geschlossenen Zustand des Instruments (siehe Fig. 5) aneinander anliegen und einen einzuklemmenden Gegenstand, beispielsweise ein OP-Tuch, fixieren. Die Griffabschnitte 40 der beiden Branchen 2, 4 weisen an ihren proximalen Enden jeweils eine Grifföse 42 auf, über die der Anwender das Instrument 1 nach Art einer Schere greifen und bedienen kann. Unmittelbar distal zu den Griffösen 42 weist jede Branche 2, 4 auf der Instrumenteninnenseite jeweils eine Zunge 44 mit drei hintereinander angeordneten Rasthaken 46 auf, die sich im geschlossenen Zustand des Instruments 1 einander gegenüberliegen und ineinander greifen, um die Branchen 2, 4 des Instruments 1 aneinander zu halten. Die Rasthaken 46 weisen jeweils eine schräge Abgleitfläche und eine sich im Wesentlichen senkrecht zur Längserstreckung der Zunge 44 emporhebende Rastfläche auf. Zum Schließen des auf diese Weise gebildeten Rastschlusses werden die Branchen 2, 4 manuell aufeinander zu gedrückt und die Rasthaken 46 gleiten entlang ihrer schrägen Abgleitflächen aneinander vorbei. Dabei stellt jede Position hinter den Rastflächen jeweils eine Schließposition dar. Zum Öffnen des Instruments 1 werden die Branchen 2, 4 manuell aus ihrer Schwenkebene (elastisch) ausgelenkt, sodass die Rasthaken 46 außer Eingriff gebracht werden und die Branchen 2, 4 wieder voneinander weg geschwenkt werden können.

Unter der Instrumenteninnenseite sind in dem vorstehenden Zusammenhang die beiden Seiten der Branchen 2, 4 zu verstehen, die in geschlossenem Zustand des Instruments 1 einander zugewandt sind. Folglich können die beiden Seiten der Branchen 2, 4, die in geschlossenem Zustand des Instruments 1 voneinander abgewandt sind, als Instrumentenaußenseite bezeichnet werden.

In Fig. 1 ist weiter zu sehen, dass die Effektorabschnitte 38 jeweils eine Effektorinnenfläche 48 aufweisen. Unter der Effektorinnenfläche 48 ist jeweils die Fläche eines Effektorabschnitts 38 zu verstehen, die im geschlossenen Zustand des Instruments 1 jeweils dem Effektorabschnitt 38 der anderen Branche 2, 4 zugewandt ist. Wie in Fig. 1 zu erkennen ist, ist der Übergang von der Effektorinnenfläche 48 der weiblichen Branche 2 zu deren distalen Querflächen 30a am Schlussabschnitt 6 aus- bzw. abgerundet. Die Außenflächen 50 des Effektorabschnitts 38 an der weiblichen Branche 2, die voneinander abgewandt sind, verlaufen zudem bündig mit den Schlussaußenflächen 24 des weiblichen Schlussabschnitts 6. Auch mit Blick auf die männliche Branche 4 ist zu erkennen, dass der Übergang von der Effektorinnenfläche 48 der männlichen Branche 4 zu dessen distaler Querfläche 36a am Schlussabschnitt 8 abgerundet ist. Die Außenflächen 52 des Effektorabschnitts 38 an der männlichen Branche 4, die voneinander abgewandt sind, sind nicht bündig mit den Außenkontaktflächen 32, sondern springen gegenüber diesen im Wesentlichen senkrecht vor. Dadurch bilden die Außenflächen 52 des Effektorabschnitts 38 an der männlichen Branche 4 zu den Außenkontaktflächen 32 des männlichen Schlussabschnitts 8 jeweils eine von der Außenkontaktfläche 32 emporragende Stufe 54. Diese Stufe 54 kann senkrecht, aber auch schräg bzw. abgeschrägt oder als Radius ausgebildet sein. Wie in Fig. 1 zu erkennen ist, befinden sich die genannten Stufen 54 längsversetzt zur distalen Querfläche 36a des männlichen Schlussabschnitts 8, sodass die jeweils gemeinsamen Kanten der Stufen 54 und Außenkontaktflächen 32 nicht mit der gemeinsamen Kante der Effektorinnenfläche 48 und distalen Querfläche 36a des männlichen Schlussabschnitts 8 zusammenfallen, sondern gegenüber dieser distal versetzt angeordnet sind. Auf diese Weise kann eine Kerbwirkung durch zusammenfallende Kanten vermieden werden.

In Fig. 2 ist das in Fig. 1 dargestellte Instrument 1 in einer perspektivischen Rückansicht gezeigt. Zu sehen ist hier die zweite Öffnungsaußenseite 22b des Durchgangslanglochs 10 mit in Richtung zur zweiten Öffnungsaußenseite 22b hin abgeschrägten Längsendflächen 12a, b. Weiter zeigt Fig.2, dass auch der Übergang von der Griffinnenfläche 56 der weiblichen Branche 2 zu deren proximalen Querflächen 30b am Schlussabschnitt 6 aus- bzw. abgerundet ist. Die Außenflächen 58 des Griffabschnitts 40 an der weiblichen Branche 2, die voneinander abgewandt sind, verlaufen zudem bündig mit den Schlussaußenflächen 24 des weiblichen Schlussabschnitts 6. Unter der Griffinnenfläche 56 ist jeweils die Fläche eines Griffabschnitts 40 zu verstehen, die im geschlossenen Zustand des Instruments 1 jeweils dem Griffabschnitt 40 der anderen Branche 2, 4 zugewandt ist. Mit Blick auf die männliche Branche 4 ist in Fig. 2 zu sehen, dass auch der Übergang von der Griffinnenfläche 56 der männlichen Branche 4 zu dessen proximaler Querfläche 36b abgerundet ist. Auch die Außenflächen 59 des Griffabschnitts 40 an der männlichen Branche 4 sind nicht bündig mit den Außenkontaktflächen 32, sondern springen gegenüber diesen im Wesentlichen senkrecht vor. Dadurch bilden die Außenflächen 59 des Griffabschnitts 40 an der männlichen Branche 4 zu den Außenkontaktflächen 32 des männlichen Schlussabschnitts 8 jeweils eine von der Außenkontaktfläche 32 emporragende Stufe 60. Auch diese Stufe 60 kann senkrecht, aber auch schräg bzw. abgeschrägt oder als Radius ausgebildet sein. Wie schon in Bezug auf den Effektorabschnitt 38 der männlichen Branche 4 erläutert, befinden sich die genannten Stufen 60 auch längsversetzt zur proximalen Querfläche 36b des männlichen Schlussabschnitts 8, sodass die jeweils gemeinsamen Kanten der Stufen 60 und Außenkontaktflächen 32 nicht mit der gemeinsamen Kante der Griffinnenfläche 56 und proximalen Querfläche 36b des männlichen Schlussabschnitts 8 zusammenfallen, sondern gegenüber diesen proximal versetzt angeordnet sind, um eine Kerbwirkung zu vermeiden.

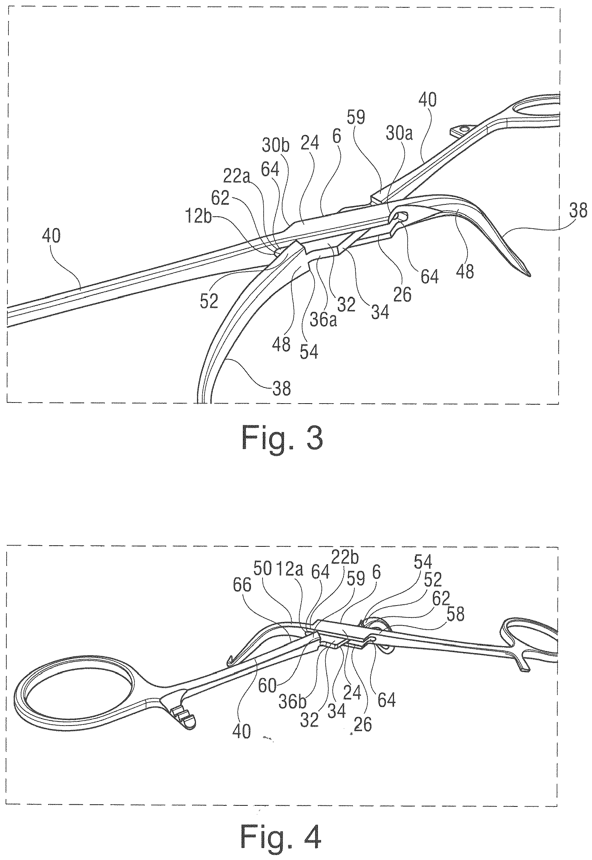

Fig. 3 zeigt die in Fig. 1 abgebildete Ausführungsform des chirurgischen Instruments 1 in einer maximal geöffneten Position aus einer perspektivischen frontalen Ansicht. Die gezeigte Relativlage der Branchen 2, 4 zueinander kann auch als vollständig geöffnete Position des Instruments 1 bzw. der Branchen 2, 4 bezeichnet werden. Wie insbesondere mit Blick auf den linken Teil der Fig. 2 deutlich wird, ist das Durchgangslangloch 10 mit einer derartigen Länge ausgeführt, dass die männliche Branche 4 am Übergang von Effektorabschnitt 38 zum männlichen Schlussabschnitt 8 mit ihrer Außenfläche, konkret mit ihrer Außenfläche 62 auf der Instrumentenaußenseite, an der ersten Öffnungsaußenseite 22a des weiblichen Schlussabschnitts 6 anliegt, ohne das Durchgangslangloch 10 an der ersten Öffnungsaußenseite 22a zu verschließen. Es besteht also zwischen der proximalen Längsendfläche 12b des Durchgangslanglochs 10 und einer Längsfläche 34 des männlichen Schlussabschnitts 6 ein durchströmbarer Kanal 64 im Schlussbereich 16 des Instruments 1.

In Fig. 4 ist die in Fig. 1 abgebildete Ausführungsform des chirurgischen Instruments 1 in einer maximal geöffneten Position aus einer perspektivischer Rückansicht gezeigt. Analog zur Anlageposition der männlichen Branche 4 an der ersten Öffnungsaußenseite 22a des weiblichen Schlussabschnitts 6 liegt die männliche Branche 4 an ihrem Übergang von Griffabschnitt 40 zum Schlussabschnitt 8 mit ihrer Außenfläche, konkret mit ihrer Außenfläche 66 auf der Instrumentenaußenseite, an der zweiten Öffnungsaußenseite 22b des weiblichen Schlussabschnitts 6 an, ohne das Durchgangslangloch 10 an der zweiten Öffnungsaußenseite 22b zu verschließen, da auch auf dieser Längsseite des Durchgangslanglochs 10 dessen Länge über die Anlageposition der männlichen Branche 4 an der Öffnungsaußenseite 22b hinausgeht und zwischen dessen distaler Längsendfläche 12a und einer Längsfläche 34 des männlichen Schlussabschnitts 8 ein durchströmbarer Kanal 64 im Schlussbereich 16 des Instruments 1 gebildet ist.

In Fig. 5 ist eine Draufsicht auf das in Fig. 1 gezeigte chirurgisches Instrument 1 in vollständig geschlossenem Zustand dargestellt. In diesem Zustand sind die beiden Branchen 2, 4 maximal miteinander verrastet, d. h. alle drei Rasthaken 46 an jeweils einer Zunge 44 der beiden Griffabschnitte 40 befinden sich mit den Rasthaken 46 der jeweils anderen Zunge 44 in Eingriff. Dabei befinden sich die Effektorabschnitt 38 der beiden Branchen 2, 4 unmittelbar distal vom Schlussbereich 16 des Instruments 1 in Anlage, ebenso liegen die Griffabschnitte 40 der beiden Branchen 2, 4 unmittelbar proximal vom Schlussbereich 16 des Instruments 1 aneinander an. In dieser Ansicht ist zu erkennen, dass die Stufen 54, 60 sowohl am Effektorabschnitt 38, als auch am Griffabschnitt 40 der männlichen Branche 4 zu den Querkanten bzw. Querflächen 30a, b des weiblichen Schlussabschnitts 6 in Instrumentenlängsrichtung zueinander beabstandet sind und im Wesentlichen parallel zueinander verlaufen. Es sei angemerkt, dass in Fig. 5 zwar nur die Draufsicht auf den Schlussbereich 16 des Instruments 1 gezeigt ist, der Schlussbereich 16 auf seiner Unterseite aber eine funktional gleiche, wenn auch spiegelbildliche, Geometrie bzw. Anlageposition der Branchen 2, 4 aneinander zeigt.

Fig. 6 zeigt schließlich eine Seitenansicht des in Fig. 1 gezeigten chirurgischen Instruments 1 in vollständig geschlossenem Zustand. Zu sehen ist wieder, dass die Stufen 54 von den Außenkontaktflächen 32 des männlichen Schlussabschnitts 8 zum Effektorabschnitt 38 der männlichen Branche 4 zu den distalen Querkanten bzw. Querflächen 30a des weiblichen Schlussabschnitts 6 beabstandet sind. Die Außenkontaktflächen 32 des männlichen Schlussabschnitts 8 liegen an den Innenkontaktflächen 18 des Durchgangslanglochs 10 des weiblichen Schlussabschnitts 6 an. Zu sehen ist weiter, dass auch in vollständig geschlossenem Zustand ein Kanal 64 zwischen der proximalen Längsendfläche 12b des Durchgangslanglochs 10 und der proximalen Querfläche 36b des männlichen Schlussabschnitts 8 vorhanden ist, der von einem Fluid, insbesondere Reinigungsfluid durchströmbar ist. Auch wenn in Fig. 6 nur der Kanal 64 zwischen proximaler Längsendfläche 12b am weiblichen Schlussabschnitt 6 und proximaler Querfläche 36b am männlichen Schlussabschnitt 8 und nur der Abstand zwischen den Stufen 54 zum Effektorabschnitt 38 und den distalen Querflächen 30a des weiblichen Schlussabschnitts 6 gezeigt ist, versteht es sich aufgrund der symmetrischen Geometrie der Branchen 2, 4, dass auch auf der nicht gezeigten Seitenansicht des chirurgischen Instruments 1 sowohl ein Kanal 64 zwischen der distalen Längsendfläche 12a am weiblichen Schlussabschnitt 6 und der distalen Querfläche 36a am männlichen Schlussabschnitt 8, als auch ein Abstand zwischen den Stufen 30 zum Griffabschnitt 40 und den proximalen Querflächen 30b des weiblichen Schlussabschnitts 8 vorhanden sind.

Zur Innengeometrie des Durchgangslanglochs 10 ist weiter anzumerken, dass diese die Form zweier verschnittener Teilkreise einnimmt. Konkret wird das Durchgangslangloch 10 hergestellt, indem zunächst an einer der beiden Öffnungsaußenseiten 22a, b ein teilkreisförmiges Volumen ausgetragen wird, beispielsweise durch Fräsen, und dann von der anderen Öffnungsaußenseite 22a, b her ebenfalls ein teilkreisförmiges Volumen ausgetragen wird, sodass es zwischen den beiden Öffnungsaußenseiten 22a, b zu einem Durchbruch in der Form eines Langlochs kommt, dessen Längsendflächen 12a, b ein in Längsrichtung der Branche 2 Teilkreisrundes Profil haben und schräg zu den Öffnungsaußenseiten 22a, b verlaufen.

### Bezugszeichenliste

- 1: Chirurgisches Instrument
- 2: Weibliche Branche
- 4: Männliche Branche
- 6: Weiblicher Schlussabschnitt
- 8: Männliches Schlussabschnitt
- 10: Durchgangsöffnung / Durchgangslangloch
- 12a: Distale Längsendfläche der Durchgangsöffnung
- 12b: Proximale Längsendfläche der Durchgangsöffnung
- 14a: Außenkante der distalen Längsendfläche zur ersten Öffnungsaußenseite
- 14b: Außenkante der distalen Längsendfläche zur zweiten Öffnungsaußenseite
- 14c: Außenkante der proximalen Längsendfläche zur ersten Öffnungsaußenseite
- 14d: Außenkante der proximalen Längsendfläche zur zweiten Öffnungsaußenseite
- 16: Schlussbereich des Instruments
- 18: Innenkontaktflächen
- 20a: Distales Längsende der Durchgangsöffnung
- 20b: Proximales Längsende der Durchgangöffnung
- 22a: erste Öffnungsseite
- 22b: zweite Öffnungsseite
- 24: Schlussaußenflächen
- 26: Längskanten der Schlussaußenflächen
- 28a: Distale Querkante der Schlussaußenfläche
- 28b: Proximale Querkante der Schlussaußenfläche
- 30a: Distale Querfläche des weiblichen Schlussabschnitts
- 30b: Proximale Querfläche des weiblichen Schlussabschnitts
- 32: Außenkontaktflächen
- 34: Längsflächen des männlichen Schlussabschnitts
- 36a: Distale Querfläche des männlichen Schlussabschnitts
- 36b: Proximale Querfläche des männlichen Schlussabschnitts
- 38: Effektorabschnitt
- 40: Griffabschnitt
- 42: Grifföse
- 44: Zunge
- 46: Rasthaken
- 48: Effektorinnenfläche
- 50: Außenflächen des Effektorabschnitts an der weiblichen Branche
- 52: Außenflächen des Effektorabschnitts an der männlichen Branche
- 54: Stufe von Außenkontaktfläche zu Effektorabschnitt
- 56: Griffinnenfläche
- 58: Außenfläche des Griffabschnitts an der weiblichen Branche
- 59: Außenfläche des Griffabschnitts an der männlichen Branche
- 60: Stufe von Außenkontaktfläche zu Griffabschnitt
- 62: Außenfläche des Effektorabschnitts auf der Instrumentenaußenseite
- 64: Kanal
- 66: Außenfläche des Griffabschnitts auf der Instrumentenaußenseite

## Patentansprüche

1. Chirurgisches Instrument (1) der branchenüberkreuzten Bauart mit einer weiblichen Branche (2) und einer männlichen Branche (4), die über ihre Längserstreckung jeweils einen Schlussabschnitt (6, 8) aufweisen und der Schlussabschnitt (6) der weiblichen Branche (2) von einer Durchgangsöffnung (10) durchzogen ist, die eine distale und eine proximale Längsendfläche (12a, b) mit jeweils zwei Außenkanten (14a-d) aufweist und dazu angepasst ist, den Schlussabschnitt (8) der männlichen Branche (4) aufzunehmen, sodass die beiden Schlussabschnitte (6, 8) in einer Montageposition des Instruments (4) einen Durchsteckschluss bilden, wobei
die Durchgangsöffnung (10) eine Länge hat, bei der die Außenkanten (14a-d) der Längsendflächen (12a, b) in allen Relativpositionen der beiden Branchen (2, 4), insbesondere in einer vollständig geschlossenen Position und/oder in einer vollständig geöffneten Position des Instruments (1), freiliegen und nicht von der männlichen Branche (4) verdeckt sind, **dadurch gekennzeichnet, dass**
die Durchgangsöffnung (10) weiter zwei einander gegenüberliegende Innenkontaktflächen (18) ausbildet, die die beiden Längsendflächen (12a, b) miteinander verbinden, der Schlussabschnitt (8) der männlichen Branche (4) zwei voneinander abgewandte Außenkontaktflächen (32) sowie die beiden Außenkontaktflächen (32) miteinander verbindende Außenquerflächen (34, 36a, b) ausbildet, sodass im Durchsteckschluss jeweils eine Innenkontaktfläche (18) und eine Außenkontaktfläche (32) drehbar aneinander gehalten sind, und in allen Relativpositionen der Branchen (2, 4) jeweils zwischen der proximalen und der distalen Längsendfläche (12a, b) und einer Außenquerfläche ( 34, 36a, b) ein durchströmbarer Kanal (64) gebildet ist.

2. Chirurgisches Instrument (1) nach Anspruch 1, **dadurch gekennzeichnet, dass** die Durchgangsöffnung (10) die Form eines Durchgangslanglochs einnimmt.

3. Chirurgisches Instrument nach Anspruch 1 oder 2, **dadurch gekennzeichnet, dass** die Durchgangsöffnung (10) zwei Öffnungsaußenseiten (22a, b) hat und die proximale und die distale Längsendfläche (12a, b) jeweils zumindest zu einer Öffnungsaußenseite (22a, b), bevorzugt jeweils zu beiden Öffnungsseiten (22a, b), abgeschrägt sind, um einen Öffnungsquerschnitt an zumindest der einen Öffnungsaußenseite (22a, b) zu vergrößern.

4. Chirurgisches Instrument (1) nach einem der Ansprüche 1 bis 3, **dadurch gekennzeichnet, dass** die beiden Schlussabschnitte (6, 8) der beiden Branchen (2, 4) jeweils zwischen einem distalen Effektorabschnitt (38) und einem proximalen Griffabschnitt (40) angeordnet sind, die Effektorabschnitte (38) der beiden Branchen (2, 4) jeweils eine der anderen Branche (2, 4) zugewandte Effektorinnenfläche (48) und die Griffabschnitte (40) der beiden Branchen (2, 4) jeweils eine der anderen Branche (2, 4) zugewandte Griffinnenfläche (56) aufweisen, die Außenquerflächen (34, 36a, b) des Schlussabschnitts (8) der männlichen Branche (4) eine distale Querfläche (36a), eine proximale Querfläche (36b) und zwei im Wesentlichen parallel zueinander ausgerichtete Seitenflächen (34) sind, die distale Querfläche (36a) sich an die Effektorinnenfläche (48) der männlichen Branche (4) anschließt und die proximale Querfläche (36b) sich an die Griffinnenfläche (56) der männlichen Branche (4) anschließt und die Übergange von distaler Querfläche (36a) zur Effektorinnenfläche (48) sowie von proximaler Querfläche (36b) zur Griffinnenfläche (56) in Form einer distalen bzw. proximalen Ausrundung gebildet sind.

5. Chirurgisches Instrument (1) nach Anspruch 4, **dadurch gekennzeichnet, dass** der Effektorabschnitt (38) und der Griffabschnitt (40) der männlichen Branche (4) jeweils zwei voneinander abgewandte Außenflächen (52, 59), aufweisen, die sich an die Außenkontaktflächen (32) des Schlussbereichs (8) anschließen und an ihren Übergängen zu den Außenkontaktflächen (32) jeweils eine Stufe (54, 60) ausbilden, die gegenüber der Außenkontaktfläche (32) vorspringt, wobei die Stufen (54) auf Seite der Effektorinnenfläche (48) distal zur distalen Ausrundung und die Stufen (60) auf Seite der Griffinnenfläche (56) proximal zur proximalen Ausrundung angeordnet sind.

6. Chirurgisches Instrument (1) nach Anspruch 5, **dadurch gekennzeichnet, dass** der Schlussabschnitt (6) der weiblichen Branche (2) zwei im Wesentlichen parallel zu den Innenkontaktflächen (18) verlaufende Schlussaußenflächen (24) aufweist, die im geschlossenen Zustand des Instruments (1) zu den Außenflächen (52, 59) des Griffabschnitts (40) sowie des Effektorabschnitts (38) der männlichen Branche (4) beabstandet angeordnet sind.

## Claims

1. A surgical instrument (1) of cross-branched design having a female branch (2) and a male branch (4), each having a terminal portion (6, 8) along their longitudinal extent, and the terminal portion (6) of the female branch (2) being pierced by a passage opening (10) having a distal and a proximal longitudinal end surface (12a, b), each having two outer edges (14a-d) and being adapted to receive the terminal portion (8) of the male branch (4), so that the two terminal portions (6, 8) form a box-lock mechanism in an assembly position of the instrument (4), wherein
the passage opening (10) has a length at which the outer edges (14a-d) of the longitudinal end surfaces (12a, b) are exposed and are not covered by the male branch (4) in all relative positions of the two branches (2, 4), in particular in a fully closed position and/or in a fully open position of the instrument (1), **characterized in that**
the passage opening (10) further forms two opposite inner contact surfaces (18) connecting the two longitudinal end surfaces (12a, b), the terminal portion (8) of the male branch (4) forms two outer contact surfaces (32) facing away from each other and outer transverse surfaces (34, 36a, b) connecting the two outer contact surfaces (32) to each other, so that in the box-lock mechanism in each case an inner contact surface (18) and an outer contact surface (32) are held rotatably against each other, and in all relative positions of the branches (2, 4) in each case between the proximal and the distal longitudinal end surface (12a, b) and an outer transverse surface (34, 36a, b), a channel (64) through which flow is possible is formed.

2. The surgical instrument (1) according to claim 1, **characterized in that** the passage opening (10) takes the form of an elongated passage hole.

3. The surgical instrument according to claim 1, **characterized in that** the passage opening (10) has two outer sides (22a, b) of the opening and the proximal and the distal longitudinal end surface (12a, b) are each beveled at least to one outer side of the opening (22a, b), preferably to both opening sides (22a, b), in order to increase an opening cross-section at at least the one outer side of the opening (22a, b).

4. The surgical instrument (1) according to one of claims 1 to 3, **characterized in that** the two terminal portions (6, 8) of the two branches (2, 4) are each arranged between a distal effector portion (38) and a proximal handle portion (40), wherein the effector portions (38) of the two branches (2, 4) each have an inner effector surface (48) facing the other branch (2, 4) and the handle portions (40) of the two branches (2, 4) each have an inner handle surface (56) facing the other branch (2, 4), the outer transverse surfaces (34, 36a, b) of the terminal portion (8) of the male branch (4) are a distal transverse surface (36a), a proximal transverse surface (36b), and two side surfaces (34) oriented substantially parallel to each other, wherein the distal transverse surface (36a) adjoins the inner effector surface (48) of the male branch (4) and the proximal transverse surface (36b) adjoins the inner handle surface (56) of the male branch (4), and the transitions from the distal transverse surface (36a) to the inner effector surface (48) as well as from the proximal transverse surface (36b) to the inner handle surface (56) are formed in the form of a distal or respectively proximal rounding.

5. The surgical instrument (1) according to claim 4, **characterized in that** the effector portion (38) and the handle portion (40) of the male branch (4) each have two outer surfaces (52, 59) facing away from each other, which adjoin the outer contact surfaces (32) of the terminal region (8) and, at their transitions to the outer contact surfaces (32), they each form a step (54, 60) which protrudes with respect to the outer contact surface (32), wherein the steps (54) on the side of the inner effector surface (48) are arranged distal to the distal rounding and the steps (60) on the side of the inner handle surface (56) are arranged proximal to the proximal rounding.

6. The surgical instrument (1) according to claim 5, **characterized in that** the terminal portion (6) of the female branch (2) has two outer terminal surfaces (24) extending substantially parallel to the inner contact surfaces (18), which, in the closed state of the instrument (1), are arranged at a distance from the outer surfaces (52, 59) of the handle portion (40) and of the effector portion (38) of the male branch (4).

## Revendications

1. Instrument chirurgical (1) de construction croisée entre branches avec une branche femelle (2) et une branche mâle (4) qui présentent sur leur étendue longitudinale respectivement une section finale (6, 8) et la section finale (6) de la branche femelle (2) est traversée par une ouverture de passage (10) qui présente une surface d'extrémité longitudinale distale et une surface d'extrémité longitudinale proximale (12a, b) avec respectivement deux bords extérieurs (14a-d) et est adaptée pour recevoir la section finale (8) de la branche mâle (4), de sorte que les deux sections finales (6, 8) forment un mécanisme de fermeture de sécurité dans une position de montage de l'instrument (4), dans lequel
l'ouverture de passage (10) possède une longueur pour laquelle les côtés extérieurs (14a-d) des surfaces d'extrémité longitudinales (12a, b) sont exposés dans toutes les positions relatives des deux branches (2, 4), en particulier dans une position entièrement fermée et/ou dans une position entièrement ouverte de l'instrument (1), et ne sont pas recouverts par la branche mâle (4), **caractérisé en ce que**
l'ouverture de passage (10) forme en outre deux surfaces de contact intérieures (18) opposées l'une à l'autre qui relient les deux surfaces d'extrémité longitudinales (12a, b) entre elles, la section finale (8) de la branche mâle (4) forme deux surfaces de contact extérieures (32) éloignées l'une de l'autre, ainsi que les surfaces transversales extérieures (34, 36a, b) reliant les deux surfaces de contact extérieures (32) l'une à l'autre, de sorte que dans le mécanisme de fermeture de sécurité respectivement une surface de contact intérieure (18) et une surface de contact extérieure (32) sont maintenues ensemble en rotation, et dans toutes les positions relatives des branches (2, 4) un canal pouvant être traversé est formé respectivement entre la surface d'extrémité longitudinale proximale et la surface d'extrémité longitudinale distale (12a, b) et une surface transversale extérieure (34, 36a, b).

2. Instrument chirurgical (1) selon la revendication 1, **caractérisé en ce que** l'ouverture de passage (10) prend la forme d'un trou oblong de passage.

3. Instrument chirurgical selon la revendication 1 ou 2, **caractérisé en ce que** l'ouverture de passage (10) possède deux côtés extérieurs d'ouverture (22a, b) et la surface d'extrémité longitudinale proximale et la surface d'extrémité longitudinale distale (12a, b) sont chanfreinées respectivement au moins par rapport à un côté extérieur d'ouverture (22a, b), de préférence respectivement par rapport aux deux côtés d'ouverture (22a, b) pour agrandir une section transversale d'ouverture au niveau d'au moins un côté extérieur d'ouverture (22a, b).

4. Instrument chirurgical (1) selon l'une quelconque des revendications 1 à 3, **caractérisé en ce que** les deux sections finales (6, 8) des deux branches (2, 4) sont disposées respectivement entre une section d'effecteur distale (38) et une section de préhension proximale (40), les sections d'effecteur (38) des deux branches (2, 4) présentent respectivement une surface intérieure d'effecteur (48) tournée vers l'autre branche (2, 4) et les sections de préhension (40) des deux branches (2, 4) présentent respectivement une surface intérieure de préhension (56) tournée vers l'autre branche (2, 4), les surfaces transversales extérieures (34, 36a, b) de la section finale (8) de la branche mâle (4) sont une surface transversale distale (36a), une surface transversale proximale (36b) et deux surfaces latérales orientées sensiblement de manière parallèle l'une à l'autre, la surface transversale distale (36a) se raccorde à la surface intérieure d'effecteur (48) de la branche mâle (4) et la surface transversale proximale (36b) se raccorde à la surface intérieure de préhension (56) de la branche mâle (4) et les transitions de la surface transversale distale (36a) à la surface intérieure d'effecteur (48), ainsi que de la surface transversale proximale (36b) à la surface intérieure de préhension (56) sont formées sous la forme d'un arrondi distal ou proximal.

5. Instrument chirurgical (1) selon la revendication 4, **caractérisé en ce que** la section d'effecteur (38) et la section de préhension (40) de la branche mâle (4) présentent respectivement deux surfaces extérieures (52, 59) éloignées l'une de l'autre, qui se raccordent aux surfaces de contact extérieures (32) de la zone finale (8) et forment au niveau de leurs transitions vers les surfaces de contact extérieures (32) respectivement un niveau (54, 60) qui fait saillie par rapport à la surface de contact extérieure (32), dans lequel les niveaux (54) du côté de la surface intérieure d'effecteur (48) sont disposés de manière distale par rapport à l'arrondi distal et les niveaux (60) du côté de la surface intérieure de préhension (56) sont disposés de manière proximale par rapport à l'arrondi proximal.

6. Instrument chirurgical (1) selon la revendication 5, **caractérisé en ce que** la section finale (6) de la branche femelle (2) présente deux surfaces extérieures finales (24) s'étendant sensiblement parallèlement aux surfaces de contact intérieures (18) qui, à l'état fermé de l'instrument (1) sont disposées à distance des surfaces extérieures (52, 59) de la section de préhension (40), ainsi que de la section d'effecteur (38) de la branche mâle (4).
